(19) Europäisches Patentamt
European Patent Office
Office européen des brevets

(11) EP 0 710 476 B1

(12) # FASCICULE DE BREVET EUROPEEN

(45) Date de publication et mention de la délivrance du brevet:
**04.03.1998 Bulletin 1998/10**

(51) Int. Cl.$^6$: **A61K 7/13**

(21) Numéro de dépôt: **95402113.5**

(22) Date de dépôt: **19.09.1995**

(54) **Composition tinctoriale à base de colorants d'oxydation et procédé de teinture mettant en oeuvre cette composition**

Färbemittel mit Oxydationsfarbstoffen und Färbeverfahren mit demselben Mittel

Composition based on oxydation dyes and dye process using said composition

(84) Etats contractants désignés:
**DE ES FR GB IT**

(30) Priorité: **02.11.1994 FR 9413081**

(43) Date de publication de la demande:
**08.05.1996 Bulletin 1996/19**

(73) Titulaire: **L'OREAL**
**75008 Paris (FR)**

(72) Inventeurs:
- **Audousset, Marie-Pascale**
**F-92600 Asnieres (FR)**
- **Maubru, Mireille**
**F-78400 Chatou (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 008 039**
**DE-A- 3 743 769**
**EP-A- 0 459 901**
**FR-A- 2 364 888**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.15.12/3.4

## Description

La présente invention est relative à des compositions tinctoriales pour la coloration d'oxydation des fibres kératiniques, en particulier des fibres kératiniques humaines telles que les cheveux, contenant des colorants d'oxydation particuliers et qui sont destinées à être utilisées en association avec une composition oxydante.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols et les métadiphénols.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire à un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Parmi les précurseurs de colorants d'oxydation classiquement utilisés à ce jour, figure la paraphénylènediamine qui, souvent associée à l'orthoaminophénol (base complémentaire) et à des coupleurs dont en particulier la résorcine, permet d'obtenir des nuances profondes et intenses.

Cependant, l'utilisation de la paraphénylènediamine semble, depuis quelque temps, être remise en question, notamment pour des raisons toxicologiques.

Il a également été proposé, notamment dans les demandes de brevets DE-A-3 743 769, EP-A-0 459 901 et FR-A-2 364 888 l'utilisation de certains dérivés de paraphénylènediamine comme la 2,3-diméthyl paraphénylènediamine, la 2,6-diméthyl paraphénylènediamine ou de paraphénylènediamines substituées en position, à titre de base d'oxydation, en association avec des coupleurs classiquement utilisés en teinture d'oxydation. Il est également connu, notamment par la demande de brevet EP-A-0 008 039, que le 6-amino 3-méthyl phénol peut être utilisé pour remplacer les colorants directs qui sont utilisés en coloration d'oxydation.

La présente invention vise à proposer de nouvelles compositions pour la coloration d'oxydation des fibres kératiniques et en particulier des fibres kératiniques humaines telles que les cheveux qui, tout étant exemptes de paraphénylènediamine, présentent néanmoins de très bonnes propriétés tinctoriales.

Ainsi, la demanderesse vient maintenant de découvrir qu'il est possible d'obtenir de nouvelles teintures non toxiques et résistantes, qui engendrent des colorations intenses et peu sélectives, en associant au moins une paraphénylènediamine 2-substituée particulière et telle que définie ci-après, avec du 3-méthyl 6-aminophénol.

Cette découverte est à la base de la présente invention.

L'invention a donc pour objet une composition tinctoriale pour la coloration d'oxydation des fibres kératiniques et en particulier des fibres kératiniques humaines telles que les cheveux, qui est caractérisée par le fait qu'elle contient, dans un milieu approprié pour la teinture :

- au moins un dérivé de paraphénylènediamine, à titre de première base d'oxydation, choisi parmi la 2,6-diméthyl paraphénylènediamine, la 2,3-diméthyl paraphénylènediamine et les paraphénylènediamines de formule (I) suivante :

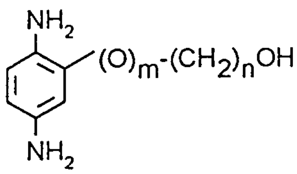

(I)

dans laquelle m est un nombre entier égal à zéro ou 1, et n est un nombre entier compris entre 1 et 4 inclusivement, ainsi que les sels d'addition de ces composés avec un acide.

- et du 3-méthyl 6-aminophénol et/ou l'un de ses sels d'addition avec un acide, à titre de deuxième base d'oxydation.

Les compositions selon l'invention sont faiblement sélectives et les colorations obtenues présentent par ailleurs une bonne puissance tinctoriale et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition.

Les sels d'addition avec un acide utilisables dans le cadre des compositions tinctoriales de l'invention sont notamment choisis parmi les chlorhydrates, les bromhydrates, les sulfates et les tartrates.

Parmi les paraphénylènediamines de formule (I) ci-dessus, on peut plus particulièrement citer la 2-(β-hydroxyméthyl) paraphénylènediamine, la 2-(β-hydroxyéthyl) paraphénylènediamine et la 2-(β-hydroxyéthoxy) paraphénylènediamine ainsi que leurs sels.

Selon un mode de réalisation particulièrement préféré de l'invention, les dérivés de paraphénylènediamine sont choisis parmi la 2,6-diméthyl paraphénylènediamine, la 2-(β-hydroxyéthyl) paraphénylènediamine et leurs sels.

L'ensemble des bases d'oxydation conformes à l'invention, c'est à dire le ou les dérivés de paraphénylènediamine et le 3-méthyl 6-aminophénol, représente de préférence de 0,01 à 10 % en poids environ du poids total de la composition tinctoriale et encore plus préférentiellement de 0,05 à 5 % en poids environ.

Les compositions tinctoriales de l'invention peuvent également contenir au moins un coupleur choisi parmi les coupleurs classiquement employés en coloration d'oxydation, et parmi lesquels on peut citer les méta-diphénols, les métaaminophénols, les métaphénylènediamines, les composés hétérocycliques tels que les pyrazolones et les dérivés d'indole.

A titre d'exemples de coupleurs utilisables dans le cadre de la présente invention, on peut plus particulièrement citer, la résorcine, la 2-méthylrésorcine, le métaaminophénol, le 2-méthyl 5-aminophénol, le 2-méthyl 5-N-(β-hydroxyéthyl aminophénol, le 2,6-diméthyl 3-aminophénol, le 2,4-diamino 1,3-diméthoxybenzène, le 2,4-diaminoanisole, le 2,4-diaminophénoxyéthanol, le 2-N-(β-hydroxyéthyl)amino 4-aminophénoxyéthanol, le 2-amino 4-N-(β-hydroxyéthyl)amino anisole, la 2,4-diaminophénoxyéthylamine, la 6-hydroxybenzomorpholine, la 6-amino benzomorpholine, le 3,4-méthylènedioxyphénol, la 3,4-méthylènedioxyaniline, le 6-hydroxyindole, le 7-aminoindole, et leurs sels d'addition avec un acide.

Ces coupleurs, lorsqu'ils sont utilisés, représentent généralement de 0,005 à 10% en poids environ du poids total de la composition tinctoriale, et de préférence de 0,01 à 5 % en poids environ.

La composition tinctoriale selon l'invention peut en outre renfermer au moins un colorant direct destiné à enrichir en reflets les nuances obtenues. Ces colorants directs peuvent par exemple être choisis parmi les colorants azoïques, anthraquinoniques et les dérivés nitrés de la série benzénique.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale telle que définie précédemment est généralement compris entre 3 et 11. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

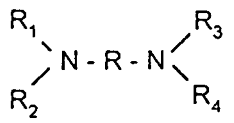

(II)

dans laquelle R est un reste propylène éventuellement substitué par un groupement hydroxyle ou un radical alkyle en $C_1$-$C_4$ ; $R_1$, $R_2$, $R_3$ et $R_4$, identiques ou différents, représentent un atome d'hydrogène, un radical alkyle en $C_1$-$C_4$ ou hydroxyalkyle en $C_1$-$C_4$.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 5 à 40 minutes environ, de préférence 15 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 11 environ et encore plus préférentiellement entre 5 et 10. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**<u>EXEMPLES 1 A 6</u>**

On a préparé les compositions tinctoriales suivantes (teneurs en grammes) :

| EXEMPLE | 1 (*) | 2 (*) | 3 (*) | 4 | 5 (*) | 6 |
|---|---|---|---|---|---|---|
| Paraphénylènediamine | 0,108 | 0,108 | | | | |
| Dichlorhydrate de 2-(β-hydroxyéthyl) paraphénylènediamine | | | 0,225 | **0,225** | | |
| Dichlorhydrate de 2,6-diméthyl para-phénylènediamine | | | | | 0,209 | **0,209** |
| Orthoaminophénol | 0,436 | | 0,436 | | 0,436 | |
| 3-méthyl 6-aminophénol | | 0,493 | | **0,493** | | **0,493** |
| Résorcine | 0,550 | 0,550 | 0,550 | **0,550** | 0,550 | **0,550** |
| Support de teinture commun (**) | 60,94 | 60,94 | 60,94 | **60,94** | 60,94 | **60,94** |
| Eau déminéralisée q.s.p. | 100 g | 100 g | 100 g | **100 g** | 100 g | **100 g** |

(*) : ces compositions tinctoriales ne font pas partie de l'invention.

(**) Support de teinture commun :

- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 ® par la société AKZO 7,0 g
- Laurylamino succinamate de diéthylaminopropyle, sel de sodium, à 55 % de M.A. 3,0 g M.A.
- Alcool oléique 5,0 g
- Diéthanolamide d'acide oléique 12,0 g

| | | |
|---|---|---|
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, chaque composition tinctoriale a été mélangée poids pour poids avec une composition oxydante constituée d'une solution de peroxyde d'hydrogène à 20 volumes (6 % en poids) et de pH 3.

Chaque mélange résultant a été ensuite appliqué pendant 30 minutes sur une mèche de cheveux gris naturels à 90 % de blancs (mèche n° 1 de cheveux non sensibilisés), et sur une mèche de ces mêmes cheveux gris à 90 % de blancs mais ayant subi une permanente (mèche n° 2 de cheveux sensibilisés). Les cheveux ont ensuite été rincés, lavés avec un shampooing standard et séchés.

La couleur des mèches a ensuite été évaluée dans le système MUNSELL au moyen d'un colorimètre CM 2002 MINOLTA ®.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur entre deux mèches est calculée en appliquant la formule de NICKERSON : $\Delta E = 0{,}4\ Co\Delta H + 6\Delta V + 3\Delta C$ , telle que décrite par exemple dans "Couleur, Industrie et Technique"; pages 14-17 vol. n° 5; 1978.

Dans cette formule, $\Delta E$ représente la différence de couleur entre deux mèches, $\Delta H$, $\Delta V$ et $\Delta C$ représentent la variation en valeur absolue des paramètres H, V et C et Co représente la pureté de la mèche par rapport à laquelle on désire évaluer la différence de couleur.

Les résultats sont donnés dans le tableau ci-dessous :

| **EXEMPLES** | **Couleur sur cheveux naturels** | **Couleur sur cheveux permanentés** | **Différence de couleur** | | | |
|---|---|---|---|---|---|---|
| | | | ΔH | ΔV | ΔC | **ΔE** |
| | | | | | | |
| **1 (*)** | 2,4 Y 3,5 / 2,3 | 2,9 Y 3,3 / 2,0 | 0,5 | 0,2 | 0,3 | **2,6** |
| **2 (*)** | 0,3 Y 3,8 / 1,9 | 2,4 Y 3,6 / 2,3 | 2,1 | 0,2 | 0,4 | **4,0** |
| | | | | | | |
| **3 (*)** | 3,9 Y 4,3 / 2,8 | 3,9 Y 3,7 / 2,2 | 0 | 0,6 | 0,6 | **5,4** |
| **4** | 5,3 Y 4,4 / 2,7 | 5,5 Y 3,9 / 2,8 | 0,2 | 0,5 | 0,1 | **3,5** |
| | | | | | | |
| **5 (*)** | 4,0 Y 4,1 / 2,5 | 3,4 Y 3,5 / 2,0 | 0,6 | 0,6 | 0,5 | **5,7** |
| **6** | 5,4 Y 4,0 / 1,8 | 5,7 Y 3,6 / 2,0 | 0,3 | 0,4 | 0,2 | **3,2** |

**(*)** : Exemples ne faisant pas partie de l'invention.

Ces résultats montrent que les compositions conformes à l'invention, tout en étant exemples de paraphénylènediamine, présentent encore une faible sélectivité ou , du moins, une sélectivité acceptable.

Ces résultats montrent plus particulièrement que :

**1)** lorsque l'on remplace la paraphénylènediamine par un précurseur de colorant d'oxydation conforme à l'invention, comme par exemple le dichlorhydrate de 2-(β-hydroxyéthyl) paraphénylènediamine ou le dichlorhydrate de 2,6-diméthyl paraphénylènediamine, en lui associant de l'orthoaminophénol qui ne fait pas partie de l'invention, on observe une augmentation inacceptable de la sélectivité des colorations obtenues (comparaisons entre les exemples 1 et 3, ou 1 et 5),

**2)** lorsque l'on remplace cette fois l'orthoaminophénol par du 3-méthyl 6-aminophénol conforme à l'invention mais que l'on conserve la paraphénylènediamine, on observe également une augmentation inacceptable de la sélectivité des colorations obtenues (comparaison entre les exemples 1 et 2),

**3)** lorsque que l'on remplace à la fois (i) la paraphénylènediamine par le dichlorhydrate de 2-(β-hydroxyéthyl) paraphénylènediamine ou le dichlorhydrate de 2,6-diméthyl paraphénylènediamine conformes à l'invention, et (ii) l'orthoaminophénol par du 3-méthyl 6-aminophénol conforme à l'invention, alors on observe alors une augmentation bien moindre de la sélectivité des colorations obtenues (comparaisons des exemples 3 et 4, ou 5 et 6).

**EXEMPLES 7 et 8 :**

On a préparé les compositions tinctoriales conformes à l'invention suivantes (teneurs en grammes) :

| EXEMPLE | 7 | 8 |
|---|---|---|
| Dichlorhydrate de 2,3-diméthyl paraphénylènediamine | 0,314 | |
| Dichlorhydrate de 2-(β-hydroxyéthoxy) paraphénylènediamine | | 0,120 |
| 3-méthyl 6-aminophénol | 0,308 | 0,431 |
| 2-méthylrésorcine | 0,496 | |
| Dichlorhydrate de 2,4-diaminophénoxyéthanol | | 0,964 |
| Support de teinture commun (*) | 60,94 | 60,94 |
| Eau déminéralisée q.s.p. | 100 g | 100 g |

(*) Support de teinture commun :

- Alcool oléique polyglycérolé à 2 moles de glycérol 4,0 g
- Alcool oléique polyglycérolé à 4 moles de glycérol, à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 ® par la société AKZO 7,0 g
- Laurylamino succinamate de diéthylaminopropyle, sel de sodium, à 55 % de M.A. 3,0 g M.A.

| | | |
|---|---|---|
| - Alcool oléique | 5,0 | g |
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, chaque composition tinctoriale a été mélangée poids pour poids avec une composition oxydante constituée d'une solution de peroxyde d'hydrogène à 20 volumes (6 % en poids) et de pH 3.

Chaque mélange résultant a été ensuite appliqué pendant 30 minutes sur une mèche de cheveux gris naturels à 90 % de blancs. Les cheveux ont ensuite été rincés, lavés avec un shampooing standard et séchés.

Les résultats tinctoriaux sont donnés dans le tableau ci-dessous :

| EXEMPLE | COLORATION OBTENUE |
|---|---|
| 7 | Beige cendré mat |
| 8 | Vert légèrement doré |

**Revendications**

1. Composition tinctoriale pour la coloration d'oxydation des fibres kératiniques et en particulier des fibres kératiniques humaines telles que les cheveux, caractérisée par le fait qu'elle contient, dans un milieu approprié pour la teinture :

   - au moins un dérivé de paraphénylènediamine, à titre de première base d'oxydation, choisi parmi la 2,6-diméthyl paraphénylènediamine, la 2,3-diméthyl paraphénylènediamine et les paraphénylènediamines de formule (I) suivante :

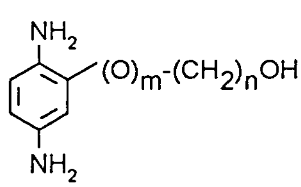

(I)

dans laquelle m est un nombre entier égal à zéro ou 1, et n est un nombre entier compris entre 1 et 4 inclusivement,
ainsi que les sels d'addition de ces composés avec un acide.

- et du 3-méthyl 6-aminophénol et/ou l'un de ses sels d'addition avec un acide, à titre de deuxième base d'oxydation.

2. Composition selon la revendication 1, caractérisée par le fait que les sels desdits dérivés de paraphénylènediamine et dudit 3-méthyl 6-aminophénol sont choisis parmi les chlorhydrates, les bromhydrates, les sulfates et les tartrates.

3. Composition selon la revendication 1 ou 2, caractérisée par le fait que lesdites paraphénylènediamines de formule (I) sont choisies parmi la 2-(β-hydroxyméthyl) paraphénylènediamine, la 2-(β-hydroxyéthyl) paraphénylènediamine, la 2-(β-hydroxyéthoxy) paraphénylènediamine et leurs sels d'addition avec un acide.

4. Composition selon la revendication 1 ou 2, caractérisée par le fait que lesdits dérivés de paraphénylènediamine sont choisis parmi la 2,6-diméthyl paraphénylènediamine, la 2-(β-hydroxyéthyl) paraphénylènediamine et leurs sels d'addition avec un acide.

5. Composition selon l'une quelconque des revendications 1 à 4, caractérisée par le fait que l'ensemble des bases d'oxydation représente de 0,01 à 10 % en poids du poids total de la composition tinctoriale.

6. Composition selon la revendication 5, caractérisée par le fait que l'ensemble des bases d'oxydation représente de 0,05 à 5 % en poids du poids total de la composition tinctoriale.

7. Composition selon l'une quelconque des revendications 1 à 6, caractérisée par le fait qu'elle contient en outre au moins un coupleur choisi parmi les métadiphénols, les méta-aminophénols, les métaphénylènediamines, les composés hétérocycliques tels que les pyrazolones et les dérivés d'indole.

8. Composition selon la revendication 7, caractérisée par le fait que ledit coupleur est choisi, seul ou en mélange, parmi la résorcine, la 2-méthylrésorcine, le métaaminophénol, le 2-méthyl 5-aminophénol, le 2-méthyl 5-N-(β-hydroxyéthyl)amino phénol, le 2,6-diméthyl 3-aminophénol, le 2,4-diamino 1,3-diméthoxybenzène, le 2,4-diaminoanisole, le 2,4-diaminophénoxyéthanol, le 2-N-(β-hydroxyéthyl)amino 4-aminophénoxyéthanol, le 2-amino 4-N-(β-hydroxyéthyl)aminoanisole, la 2,4-diaminophénoxyéthylamine, la 6-hydroxybenzomorpholine, la 6-aminobenzomorpholine, le 3,4-méthylènedioxyphénol, la 3,4-méthylènedioxyaniline, le 6-hydroxyindole, le 7-aminoindole, leurs sels d'addition avec un acide.

9. Composition selon l'une quelconque des revendications 7 à 8, caractérisée par le fait que le ou les coupleurs représentent de 0,005 à 10 % en poids du poids total de la composition tinctoriale.

10. Composition selon la revendication 9, caractérisé par le fait que le ou les coupleurs représente de 0,01 à 5 % en poids du poids total de la composition.

11. Composition selon l'une quelconque des revendications 1 à 10, caractérisée par le fait qu'elle contient en outre au moins un colorant direct.

12. Composition selon l'une quelconque des revendications 1 à 11, caractérisée par le fait que ledit milieu approprié pour la teinture est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

13. Composition selon l'une quelconque des revendications 1 à 12, caractérisée par le fait qu'elle présente un pH compris entre 3 et 11.

14. Procédé de teinture des fibres kératiniques et en particulier des fibres kératiniques humaines telles que les cheveux caractérisé par le fait que l'on applique sur ces fibres une composition tinctoriale telle que définie à l'une quelconque des revendications 1 à 13 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

15. Dispositif à plusieurs compartiments ou "kit" de teinture à plusieurs compartiments dont un premier compartiment renferme une composition tinctoriale telle que définie à l'une quelconque des revendications 1 à 13 et un second

compartiment renferme une composition oxydante.

## Claims

1. Dye composition for the oxidation dyeing of keratin fibres and in particular human keratin fibres such as the hair, which composition is characterized in that it contains, in a medium which is suitable for dyeing:

   - at least one para-phenylenediamine derivative, as first oxidation base, chosen from 2,6-dimethyl-para-phenylenediamine, 2,3-dimethyl-para-phenylenediamine and the para-phenylenediamines of formula (I) below:

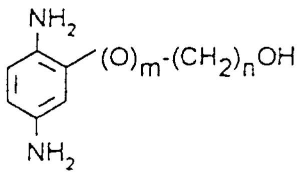

(I)

   in which m is an integer equal to zero or 1 and n is an integer between 1 and 4 inclusively, as well as the addition salts of these compounds with an acid,
   - and 3-methyl-6-aminophenol and/or one of the addition salts thereof with an acid, as second oxidation base.

2. Composition according to Claim 1, characterized in that the salts of the said para-phenylenediamine derivatives and of the said 3-methyl-6-aminophenol are chosen from the hydrochlorides, the hydrobromides, the sulphates and the tartrates.

3. Composition according to Claim 1 or 2, characterized in that the said para-phenylenediamines of formula (I) are chosen from 2-(β-hydroxymethyl)-para-phenylenediamine, 2-(β-hydroxyethyl)-para-phenylenediamine and 2-(β-hydroxyethoxy)-para-phenylenediamine, and the addition salts thereof with an acid.

4. Composition according to Claim 1 or 2, characterized in that the said para-phenylenediamine derivatives are chosen from 2,6-dimethyl-para-phenylenediamine and 2-(β-hydroxyethyl)-para-phenylenediamine, and the addition salts thereof with an acid.

5. Composition according to any one of Claims 1 to 4, characterized in that all of the oxidation bases together represent from 0.01 to 10 % by weight of the total weight of the dye composition.

6. Composition according to Claim 5, characterized in that all of the oxidation bases together represent from 0.05 to 5 % by weight of the total weight of the dye composition.

7. Composition according to any one of Claims 1 to 6, characterized in that it also contains at least one coupler chosen from meta-diphenols, meta-aminophenols, meta-phenylenediamines, and heterocyclic compounds such as pyrazolones and indole derivatives.

8. Composition according to Claim 7, characterized in that the said coupler is chosen, alone or as a mixture, from resorcine, 2-methylresorcine, meta-aminophenol, 2-methyl-5-aminophenol, 2-methyl-5-N-(β-hydroxyethyl)aminophenol, 2,6-dimethyl-3-aminophenol, 2,4-diamino-1,3-dimethoxybenzene, 2,4-diaminoanisole, 2,4-diaminophenoxyethanol, 2-N-(β-hydroxyethyl)amino-4-aminophenoxyethanol, 2-amino-4-N-(β-hydroxyethyl)aminoanisole, 2,4-diaminophenoxyethylamine, 6-hydroxybenzomorpholine, 6-aminobenzomorpholine, 3,4-methylenedioxyphenol, 3,4-methylenedioxyaniline, 6-hydroxyindole, 7-aminoindole, and the addition salts thereof with an acid.

9. Composition according to either of Claims 7 and 8, characterized in that the coupler or couplers represent from 0.005 to 10 % by weight of the total weight of the dye composition.

10. Composition according to Claim 9, characterized in that the coupler or couplers represent from 0.01 to 5 % by weight of the total weight of the composition.

11. Composition according to any one of Claims 1 to 10, characterized in that it also contains at least one direct dye.

12. Composition according to any one of Claims 1 to 11, characterized in that the said medium which is suitable for dyeing consists of water or of a mixture of water and at least one organic solvent chosen from $C_1$-$C_4$ lower alkanols, glycerol, glycols and glycol ethers, aromatic alcohols, analogous products and mixtures thereof.

13. Composition according to any one of Claims 1 to 12, characterized in that it has a pH between 3 and 11.

14. Process for dyeing keratin fibres and in particular human keratin fibres such as the hair, characterized in that a dye composition as defined in any one of Claims 1 to 13 is applied to these fibres, and in that the colour is developed at acidic, neutral or alkaline pH using an oxidizing agent which is added, only at the time of use, to the dye composition, or which is present in an oxidizing composition that is applied simultaneously or sequentially in a separate manner.

15. Multi-compartment dyeing device or "kit" containing several compartments, a first compartment of which contains a dye composition as defined in any one of Claims 1 to 13, and a second compartment of which contains an oxidizing composition.

**Patentansprüche**

1. Färbemittelzusammensetzung für die oxidative Färbung von Keratinfasern und insbesondere menschlicher Keratinfasern, wie z.B. der Haare, dadurch gekennzeichnet, daß sie in einem für die Färbung geeigneten Medium enthält:

   - mindestens ein p-Phenylendiamin-Derivat als erste Oxidationsbase, das ausgewählt ist unter 2,6-Dimethyl-p-phenylendiamin, 2,3-Dimethyl-p-phenylendiamin und p-Phenylendiaminen der folgenden Formel (I)

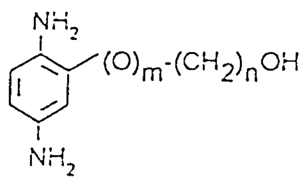

(I)

,

   in der m Null oder die ganze Zahl 1 und n eine ganze Zahl von 1 bis 4, wobei 1 und 4 eingeschlossen sind, bedeuten,
   sowie den Additionssalzen dieser Verbindungen mit einer Säure,
   - und 3-Methyl-6-aminophenol und/oder eines seiner Additionssalze mit einer Säure als zweite Oxidationsbase.

2. Zusammensetzung nach Anspruch 1, dadurch gekennzeichnet, daß die Salze der p-Phenylendiamin-Derivate und von 3-Methyl-6-aminophenol unter den Hydrochloriden, den Hydrobromiden, den Sulfaten und Tartraten ausgewählt sind.

3. Zusammensetzung nach Anspruch 1 oder 2, dadurch gekennzeichnet, daß die p-Phenylendiamine der Formel (I) unter 2-(β-Hydroxvmethyl)-p-phenylendiamin, 2-(β-Hydroxyethyl)-p-phenylendiamin und 2-(β-Hydroxyethoxy)-p-phenylendiamin sowie deren Additionssalzen mit einer Säure ausgewählt sind.

4. Zusammensetzung nach Anspruch 1 oder 2, dadurch gekennzeichnet, daß die p-Phenylendiamin-Derivate unter 2,6-Dimethyl-p-phenylendiamin, 2-(β-Hydroxyethyl)-p-phenylendiamin und deren Additionssalzen mit einer Säure ausgewählt sind.

5. Zusammensetzung nach einem der Ansprüche 1 bis 4, dadurch gekennzeichnet, daß die Oxidationsbasen insgesamt 0,01 bis 10 Gew.-% des Gesamtgewichts der Färbemittelzusammensetzung ausmachen.

6. Zusammensetzung nach Anspruch 5, dadurch gekennzeichnet, daß die Gesamtheit der Oxidationsbasen zusammen 0,05 bis 5 Gew.-% ausmacht.

7. Zusammensetzung nach einem der Ansprüche 1 bis 6, dadurch gekennzeichnet, daß sie außerdem mindestens

einen Kuppler enthält, der unter m-Diphenolen, m-Aminophenolen, m-Phenylendiaminen und heterocyclischen Verbindungen wiePyrazolonen und Indolderivaten ausgewählt ist.

**8.** Zusammensetzung nach Anspruch 7, dadurch gekennzeichnet, daß der Kuppler allein oder im Gemisch ausgewählt ist unter Resorcin, 2-Methylresorcin, m-Aminophenol, 2-Methyl-5-aminophenol, 2-Methyl-5-N-(β-hydroxyethyl)-aminophenol, 2,6-Dimethyl-3-aminophenol, 2,4-Diamino-1,3-dimethoxybenzol, 2,4-Diaminoanisol, 2,4-Diaminophenoxyethanol, 2-N-(β-Hydroxyethyl)-amino-4-amino-phenoxyethanol, 2-Amino-4-N-(β-hydroxyethyl)-aminoanisol, 2,4-Diaminophenoxyethylamin, 6-Hydroxybenzomorpholin, 6-Aminobenzomorpholin, 3,4-Methylendioxyphenol, 3,4-Methylendioxyanilin, 6-Hydroxyindol, 7-Aminoindol und deren Additionssalze mit einer Säure.

**9.** Zusammensetzung nach einem der Ansprüche 7 und 8, dadurch gekennzeichnet, daß der oder die Kuppler 0,005 bis 10 Gew.-% des Gesamtgewichts der Färbemittelzusammensetzung ausmachen.

**10.** Zusammensetzung nach Anspruch 9, dadurch gekennzeichnet, daß der oder die Kuppler 0,01 bis 5 Gew.-% des Gesamtgewichts der Zusammensetzung ausmachen.

**11.** Zusammensetzung nach einem der Ansprüche 1 bis 10, dadurch gekennzeichnet, daß sie außerdem mindestens ein direktziehendes Färbemittel enthält.

**12.** Zusammensetzung nach einem der Ansprüche 1 bis 11, dadurch gekennzeichnet, daß das für die Färbung geeignete Medium aus Wasser oder einem Gemisch aus Wasser und mindestens einem organischen Lösungsmittel besteht, das unter niederen Alkoholen mit ein bis vier Kohlenstoffatomen ($C_1$-$C_4$), Glycerin, Glykolen und Glykolethern, aromatischen Alkoholen und ähnlichen bzw. entsprechenden Produkten sowie deren Gemische ausgewählt ist.

**13.** Zusammensetzung nach einem der Ansprüche 1 bis 12, dadurch gekennzeichnet, daß sie einen pH-Wert im Bereich von 3 bis 12 aufweist.

**14.** Verfahren zur Färbung von Keratinfasern und insbesondere menschlicher Keratinfasern wie der Haare, dadurch gekennzeichnet, daß auf diese Fasern eine Färbemittelzusammensetzung nach einem der Ansprüche 1 bis 13 aufgetragen wird, daß die Farbe bei saurem, neutralem oder alkalischem pH-Wert mit Hilfe eines Oxidationsmittels entwickelt wird, das zum Zeitpunkt des Einsatzes zu der Färbemittelzusammensetzung hinzugegeben wird oder das in einer oxidierenden Zusammensetzung enthalten ist, die gleichzeitig mit der Färbemittelzusammensetzung oder getrennt nach dem Auftragen dieser Färbemittelzusammensetzung aufgetragen wird.

**15.** Vorrichtung zur Färbung mit mehreren Abteilungen oder "Kit", von denen eine erste Abteilung die in einem der Ansprüche 1 bis 13 definierte Färbemittelzusammensetzung und eine zweite Abteilung eine oxidierende Zusammensetzung enthält.